# EUROPEAN PATENT APPLICATION

(11) **EP 4 776 306 A1**
(43) Date of publication of application: **15.07.2026**
(21) Application number: 24877561.1
(22) Date of filing: 11.10.2024
(51) Int. Cl.: G16H 50/70, G16H 50/50, G16H 50/30, G06N 3/0464, G06N 3/044, G06N 3/045, G06N 3/048, G06N 3/096, G16H 50/20

(54) **METHOD, PROGRAM, AND DEVICE FOR PREDICTING DISEASE OF PATIENT ON BASIS OF NEURAL NETWORK MODEL**

(30) Priority: 11.10.2023 KR 20230134824
(71) Applicant: Medical AI Co., Ltd., Seoul 06180 (KR)
(72) Inventor: KWON, Joonmyoung, Seoul 06180 (KR); JO, Yongyeon, Seoul 06180 (KR)
(74) Representative: Patentanwaltskanzlei Matschnig & Forsthuber OG
(86) International application number: PCT/KR2024/015423
(87) International publication number: WO 2025/080026

(57) **Abstract**

The present disclosure provides a method, a device, and a program for predicting a disease of a patient based on a neural network model. A method according to an embodiment of the present disclosure comprises: acquiring a biosignal of a subject; acquiring a first result value corresponding to a characteristic of the subject or a characteristic of the biosignal, based on the acquired biosignal and a first neural network model; acquiring a second result value corresponding to a disease of the subject, based on the acquired first result value and a second neural network model; and predicting the state of the subject based on the second result value.

## Description

### TECHNICAL FIELD

The present disclosure relates to deep learning technology in the medical field, and more particularly, to a method for predicting a potential disease of a patient using the patient's biosignals and a pre-trained deep learning model.

### Background Art

With the recent development of information and communication technology, deep learning technology is being utilized in various technical fields. In particular, deep learning technology is being variously utilized in the medical field, which has traditionally relied on specialized and limited personnel such as doctors and researchers to diagnose a patient's disease. For example, this includes inputting biosignals acquired in real-time from a patient into a pre-trained deep learning model to analyze the patient's state or predict a disease.

Meanwhile, when predicting a patient's disease from biosignals using deep learning technology, the accuracy of the prediction results may be influenced by other features latent in the biosignals. Specifically, since a deep learning model is trained to predict a specific disease, there may be other feature information latent in the biosignals that the deep learning model did not identify during the training process. However, in that such feature information may have a relationship with the result value of the deep learning model, combining the feature information and the result value of the deep learning model may further enhance the accuracy of the disease prediction results. To date, a desirable countermeasure related to this has not yet been proposed in the medical application of deep learning technology.

### Disclosure

### Technical Problem

The present disclosure is devised in response to the aforementioned background art, and an object thereof is to provide a method, a program, and a device for predicting a disease of a patient based on a neural network model.

However, the problems to be solved by the present disclosure are not limited to the aforementioned problems, and other unmentioned problems can be clearly understood from the following description.

### Technical Solution

According to an embodiment of the present disclosure for achieving the aforementioned objects, a method for predicting a disease of a patient based on a neural network model, performed by a computing device including one or more processors, comprises: acquiring a biosignal of a subject; acquiring a first result value corresponding to a characteristic of the subject or a characteristic of the biosignal, based on the acquired biosignal and a first neural network model; acquiring a second result value corresponding to a state of the subject, based on the acquired first result value and a second neural network model; and predicting the state of the subject based on the second result value.

Alternatively, the acquiring of the second result value comprises: acquiring a second result value corresponding to the state of the subject by inputting the acquired biosignal into the second neural network model; and acquiring an adjusted second result value by applying a weight corresponding to the acquired first result value to the second result value.

Alternatively, the acquiring of the second result value comprises: identifying a characteristic type of the subject and determining a sign of the weight corresponding to the acquired first result value according to the characteristic type.

Alternatively, the state of the subject may comprise left ventricular systolic dysfunction; the characteristic of the subject may comprise hypertension and chronic kidney disease of the subject; and the determining of the weight may comprise applying a positive sign to the weight corresponding to the acquired first result value when the first result value corresponds to the hypertension, and applying a negative sign to the weight corresponding to the acquired first result value when the first result value corresponds to the chronic kidney disease.

Alternatively, the acquiring of the first result value may comprise acquiring the first result value corresponding to the characteristic of the subject by inputting the acquired biosignal into the first neural network model; the acquiring of the second result value may comprises acquiring the second result value corresponding to the state of the subject by inputting the acquired first result value and the acquired biosignal into the second neural network model; and the second neural network model may be trained to output the second result value corresponding to the state of the subject based on the first result value and the biosignal.

Alternatively, the method may comprise acquiring a fourth result value corresponding to the state of the subject by inputting the acquired biosignal into a third neural network model; the acquiring of the first result value may comprise acquiring the first result value corresponding to the characteristic of the subject by inputting the acquired biosignal into the first neural network model; the acquiring of the second result value may comprise acquiring the second result value corresponding to the state of the subject by inputting the acquired first result value and the acquired fourth result value into the second neural network model; and the second neural network model may be trained to output the second result value corresponding to the state of the subject based on the fourth result value corresponding to the state of the subject, the first result value, and the biosignal.

Alternatively, the method may comprise acquiring a fifth result value corresponding to another disease related to a disease included in the state by inputting the acquired biosignal into a fourth neural network model; and the acquiring of the first result value corresponding to the characteristic of the subject may comprise acquiring the first result value corresponding to the characteristic based on the acquired biosignal and the first neural network model in response to a determination based on the fifth result value that the other disease exists.

Alternatively, the characteristic of the biosignal may comprise noise included in the acquired biosignal; the method may comprise acquiring a noise score corresponding to the noise included in the biosignal as the first result value by inputting the acquired biosignal into the first neural network model; and the acquiring of the second result value may comprise acquiring the second result value corresponding to the state of the subject by inputting the acquired noise score, the acquired first result value, and the acquired biosignal into the second neural network model.

Alternatively, the method may comprise terminating a state prediction process of the subject without acquiring the second result value when the noise score is equal to or greater than a preset value.

Alternatively, the method may comprise: adjusting a performance indicator of the second neural network model based on the acquired first result value, the performance indicator comprising at least one of a cut-off, a confidence, a sensitivity, and a specificity of the second neural network model; and acquiring the second result value corresponding to the state of the subject using the second neural network model with the adjusted performance indicator.

Alternatively, the adjusting may comprise adjusting the performance indicator of the second neural network model based on the acquired first result value and information about the environment in which the biosignal was acquired.

According to an embodiment of the present disclosure, a computing device for predicting a disease of a patient based on a neural network model comprises: a processor including one or more cores; a memory including program codes executable by the processor; and a network unit. The processor is configured to: acquire a biosignal of a subject via the network unit; acquire a first result value corresponding to a characteristic of the subject based on the acquired biosignal and a first neural network model; acquire a second result value corresponding to a state of the subject based on the acquired first result value and a second neural network model; and predict the state of the subject based on the second result value.

According to an embodiment of the present disclosure, a computer program stored in a computer-readable storage medium, when executed by one or more processors, causes operations to be performed for predicting a disease of a patient based on a neural network model. The operations comprise: acquiring a biosignal of a subject; acquiring a first result value corresponding to a characteristic of the subject based on the acquired biosignal and a first neural network model; acquiring a second result value corresponding to a state of the subject based on the acquired first result value and a second neural network model; and predicting the state of the subject based on the second result value.

### ADVANTAGEOUS EFFECTS

According to the method for predicting a disease of a patient based on a neural network model of the present disclosure, the accuracy of disease prediction may be improved, allowing for a more accurate diagnosis for the patient.

### Description of Drawings

FIG. 1 is a block diagram of a computing device according to an embodiment of the present disclosure.
FIG. 2 is a flowchart schematically illustrating a method for predicting a disease of a patient based on a neural network model according to an embodiment of the present disclosure.
FIG. 3 is a diagram illustrating the accuracy of predicting left ventricular systolic dysfunction for different characteristics of subjects, according to an embodiment of the present disclosure.
FIG. 4 is an exemplary diagram schematically illustrating a method for predicting a disease of a patient based on a neural network model according to an embodiment of the present disclosure.
FIG. 5 is an exemplary diagram schematically illustrating a method for predicting a disease of a patient by inputting a first result value and a second result value into a neural network model according to an embodiment of the present disclosure.
FIG. 6 is an exemplary diagram schematically illustrating a method for predicting a disease of a patient by inputting a first result value and an electrocardiogram (Hereinafter, ECG) signal into a second neural network model according to an embodiment of the present disclosure.
FIG. 7 is an exemplary diagram illustrating determination of whether to predict a first disease based on a prediction result of a second disease that is related to the first disease, according to an embodiment of the present disclosure.
FIG. 8 is a detailed block diagram of a computing device according to another embodiment of the present disclosure.

### Mode for Invention

Hereinafter, the present disclosure describes embodiments in detail with reference to the accompanying drawings so that a person of ordinary skill in the art to which the present disclosure pertains can easily implement the same. The embodiments presented in the present disclosure are provided to enable a person of ordinary skill in the art to use or implement the content of the present disclosure. Accordingly, various modifications to the embodiments of the present disclosure will be apparent to a person of ordinary skill in the art. That is, the present disclosure may be embodied in many different forms and is not to be construed as limited to the embodiments set forth herein.

Throughout the specification of the present disclosure, the same or similar reference numerals refer to the same or similar components. Furthermore, for clarity of description of the present disclosure, reference numerals for parts unrelated to the description of the present disclosure in the drawings may be omitted.

The term "or" as used in the present disclosure is intended to mean an inclusive "or" rather than an exclusive "or". That is, unless otherwise specified or clear from the context, "X uses A or B" is to be understood to mean any of the natural inclusive permutations. For example, if "X uses A or B" is used in the present disclosure, unless otherwise specified or clear from the context, it may be interpreted as any of the cases where X uses A, X uses B, or X uses both A and B.

The term "and/or" as used in the present disclosure is to be understood to refer to and include all possible combinations of one or more of the associated listed concepts.

The terms "comprise" and/or "comprising" as used in the present disclosure are to be understood to mean the presence of a specific feature and/or component. However, the terms "comprise" and/or "comprising" are to be understood as not precluding the presence or addition of one or more other features, other components, and/or combinations thereof.

In the present disclosure, unless otherwise specified or clear from the context to indicate a singular form, generally the singular is to be interpreted to include "one or more".

The term "N-th (where N is a natural number)" as used in the present disclosure may be understood as an expression used to distinguish components of the present disclosure from each other according to a certain standard, such as a functional perspective, a structural perspective, or for convenience of explanation. For example, in the present disclosure, components that perform different functional roles may be distinguished as a first component or a second component. However, components that are substantially the same within the technical spirit of the present disclosure but need to be distinguished for convenience of explanation may also be distinguished as a first component or a second component.

The term "acquire" as used in the present disclosure may be understood as not only receiving data through a wired/wireless communication network from an external device or system, but also generating data in an on-device form.

Meanwhile, the term "module" or "unit" as used in the present disclosure may be understood as a term referring to an independent functional unit that processes computing resources, such as a computer-related entity, firmware, software, or a portion thereof, hardware or a portion thereof, or a combination of software and hardware. A "module" or a "unit" may be a unit configured as a single element, or a unit expressed as a combination or a set of a plurality of elements. For example, in a narrow sense, a "module" or a "unit" may refer to a hardware element of a computing device or a set thereof, an application program that performs a specific function of software, a processing procedure implemented through software execution, or a set of instructions for program execution. Furthermore, in a broad sense, a "module" or a "unit" may refer to a computing device itself that constitutes a system, or an application executed on the computing device. However, the above-mentioned concepts are only examples, and the concepts of "module" or "unit" may be variously defined within the scope understandable by a person of ordinary skill in the art based on the content of the present disclosure.

The term "model" as used in the present disclosure may be understood as an abstraction for a system implemented using mathematical concepts and language to solve a specific problem, a set of software units for solving a specific problem, or a processing procedure for solving a specific problem. For example, a neural network "model" may refer to an entire system implemented as a neural network that has a problem-solving ability through learning. The neural network may have problem-solving ability by optimizing parameters that connect nodes or neurons through learning. A neural network "model" may include a single neural network or a set of neural networks in which a plurality of neural networks are combined.

The term "data" as used in the present disclosure may include "images", signals, and the like. The term "image" as used in the present disclosure may refer to multi-dimensional data composed of discrete image elements. In other words, an "image" may be understood as a term referring to a digital representation of an object that can be seen by the human eye. For example, an "image" may refer to multi-dimensional data composed of elements corresponding to pixels in a 2D image. An "image" may refer to multi-dimensional data composed of elements corresponding to voxels in a 3D image.

The foregoing description of the terms is for the purpose of aiding the understanding of the present disclosure. Therefore, it is to be noted that if the foregoing terms are not explicitly stated as limiting the content of the present disclosure, they are not used in a sense that limits the technical spirit of the content of the present disclosure.

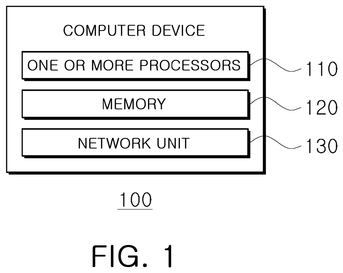

FIG. 1 is a block diagram of a computing device 100 according to an embodiment of the present disclosure.

A computing device 100 according to an embodiment of the present disclosure may be a hardware device or part of a hardware device that performs comprehensive processing and calculation of data, or it may be a software-based computing environment connected via a communication network. For example, the computing device 100 may be a server, which is a subject that performs intensive data processing functions and shares resources, or it may be a client that shares resources through interaction with a server. In addition, the computing device 100 may be a cloud system in which a plurality of servers and clients interact to process data comprehensively. The above description is only one example related to the type of the computing device 100, and therefore, the type of the computing device 100 may be variously configured within the scope understandable by a person of ordinary skill in the art based on the content of the present disclosure. For example, the computing device 100 may be implemented as various electronic devices such as a desktop, a laptop, a smartphone, a smart watch, and a server. Alternatively, the computing device 100 may be implemented as a biosignal measuring device that directly measures biosignals.

Referring to FIG. 1, a computing device 100 according to an embodiment of the present disclosure may include a processor 110, a memory 120, and a network unit 130. However, FIG. 1 is only an example, and the computing device 100 may include other components for implementing a computing environment. In addition, only some of the disclosed components may be included in the computing device 100.

The processor 110 according to an embodiment of the present disclosure may be understood as a component unit including hardware and/or software for performing computing operations. For example, the processor 110 may execute a computer program and perform data processing for machine learning. The processor 110 may process computational operations such as feature extraction for machine learning and error calculation based on backpropagation. The processor 110 for performing such data processing may include a central processing unit (CPU), a general-purpose graphics processing unit (GPGPU), a tensor processing unit (TPU), an application-specific integrated circuit (ASIC), or a field-programmable gate array (FPGA). The types of the processor 110 described above are only examples, and the type of the processor 110 may be variously configured within the scope understandable by a person of ordinary skill in the art based on the content of the present disclosure.

The processor 110 is connected to other components (i.e., the memory 120 and the network unit 130) of the computing device 100 and controls the overall operation of the computing device 100.

A memory 120 according to an embodiment of the present disclosure may be understood as a constituent unit including hardware and/or software for storing and managing data processed in a computing device 100. That is, the memory 120 may store any form of data generated or determined by the processor 110 and any form of data received by the network unit 130. For example, the memory 120 may include at least one storage medium of a flash memory type, a hard disk type, a multimedia card micro type, a card type memory, a random access memory (RAM), a static random access memory (SRAM), a read-only memory (ROM), an electrically erasable programmable read-only memory (EEPROM), a programmable read-only memory (PROM), a magnetic memory, a magnetic disk, or an optical disk. In addition, the memory 120 may include a database system that controls and manages data in a predetermined system. The foregoing types of the memory 120 are only one example, and the type of the memory 120 may be variously configured within a scope understandable by a person of ordinary skill in the art based on the content of the present disclosure.

The memory 120 may structure, organize, and manage data necessary for the processor 110 to perform operations, a combination of data, and program codes executable by the processor 110. For example, the memory 120 may store a biosignal received via a network unit 130 to be described later. In addition, the memory 120 may store a neural network model trained to predict a disease, program codes for causing training of a neural network model to be performed, program codes for causing the neural network model to receive a biosignal and perform inference in accordance with a purpose of a computing device 100, and processed data generated as the program codes are executed.

The network unit 130 according to an embodiment of the present disclosure may be understood as a constituent unit that transmits and receives data through any form of known wired/wireless communication system. For example, the network unit 130 may perform data transmission and reception using a wired/wireless communication system such as a local area network (LAN), wideband code division multiple access (WCDMA), long term evolution (LTE), wireless broadband internet (WiBro), 5th generation mobile communication (5G), ultra wide-band, ZigBee, radio frequency (RF) communication, wireless LAN, wireless fidelity (Wi-Fi), near field communication (NFC), or Bluetooth. Since the communication systems described above are only examples, the wired/wireless communication system for data transmission and reception of the network unit 130 may be variously applied in addition to the examples described above.

The network unit 130 may receive data necessary for the processor 110 to perform calculations through wired/wireless communication with any system or any client. In addition, the network unit 130 may transmit data generated through the calculation of the processor 110 through wired/wireless communication with any system or any client. For example, the network unit 130 may receive biosignals and medical data through communication with a database within a medical environment, a cloud server that performs tasks such as standardization of medical data, or a computing device. The network unit 130 may transmit output data of the neural network model, and intermediate data and processed data derived during the calculation process of the processor 110, through communication with the aforementioned database, server, or computing device.

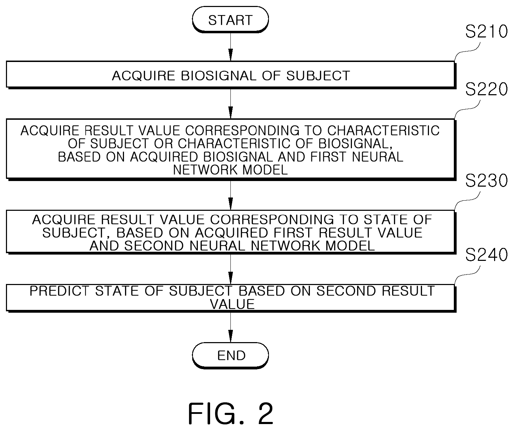

FIG. 2 is a flowchart schematically illustrating a method for predicting a disease of a patient based on a neural network model according to an embodiment of the present disclosure.

Referring to FIG. 2, the processor 110 acquires a biosignal of a subject (S310). Herein, the subject may be a subject (e.g., a patient, etc.) whose disease is to be predicted through the computing device 100.

The processor 110 may acquire the biosignal of the subject via the network unit 130. Specifically, the processor 110 may acquire the biosignal of the subject from a biosignal measuring device that is interlocked with the computing device 100 via the network unit 130. The processor 110 may further acquire biological information such as the subject's age, name, sex, height, weight, and body fat percentage, along with the biosignal of the subject.

However, the present disclosure is not limited thereto, and the processor 110 may directly acquire a biosignal of the subject through a sensor unit of the computing device 100. For example, the sensor unit may include at least one electrode, and the processor 110 may acquire an ECG signal by detecting an electrical signal generated from the heart by the at least one electrode being attached to the user's body.

Biosignals may include various types of signals that contain biological information of a subject and are acquirable from the subject. For example, the biosignals may include a user's blood pressure, ECG, electroencephalogram, body temperature, and the like.

Hereinafter, for convenience of explanation of the present disclosure, the biosignal is assumed to be an ECG signal.

According to an embodiment of the present disclosure, the processor 110 may acquire a result value corresponding to a characteristic of the subject or a characteristic of the biosignal based on the acquired biosignal and a first neural network model (S220).

Herein, the first neural network model may be a model trained to output a result value (hereinafter, first result value) corresponding to a characteristic of the subject or a characteristic of the biosignal by taking the biosignal as input. As an example, the first neural network model may comprise a model trained to output a first result value corresponding to a characteristic of the subject (a first sub-model of the first neural network model) and a model trained to output a first result value corresponding to a characteristic of the biosignal (a second sub-model of the first neural network model).

The first result value corresponding to the characteristic of the subject may be a quantified value indicating the presence or degree of the characteristic of the subject. For example, the first neural network model may be implemented as a Multi-Layer Perceptron (MLP), a Convolutional Neural Network (CNN) model, a Recurrent Neural Network (RNN) model, a Relu model, or the like.

The characteristic of the subject may be the health state of the subject or a disease different from the disease to be predicted. For example, the characteristics of the subject may include the subject's age, sex, diabetes mellitus, hypertension (HTN), chronic kidney disease (CKD), and the like. To this end, the first neural network model may be a model pre-trained based on training data comprising a plurality of input data including an ECG signal (or a feature-point-labeled ECG signal) and a plurality of output data to which a label regarding the presence or degree of hypertension (or a label regarding the presence of chronic kidney disease) is assigned.

The characteristic of the biosignal may be a degree of noise, a type of noise, or the like identified in the biosignal. To this end, the first neural network model may be a model pre-trained based on training data comprising a plurality of input data including an ECG signal (or a feature-point-labeled ECG signal) and a plurality of output data to which a noise score corresponding to the amount of noise included in each ECG signal is assigned.

The processor 110 may input the acquired biosignal into the first neural network model to acquire a first result value corresponding to a characteristic of the subject or a characteristic of the biosignal. Specifically, the first neural network model may extract feature information (e.g., feature points) related to the characteristic of the subject or the characteristic of the biosignal from the biosignal, and output a first result value corresponding to the characteristic of the subject or the characteristic of the biosignal based on the extracted feature information. For example, when the first neural network model is a model trained to determine the presence of hypertension in the subject by taking an ECG signal as input, the processor 110 may input the ECG signal into the first neural network model to acquire a first result value regarding the presence of hypertension.

The processor 110 may acquire a result value corresponding to the state of the subject based on the acquired result value corresponding to the characteristic of the subject or the characteristic of the biosignal and a second neural network model (S230).

Specifically, the processor 110 may acquire a result value (hereinafter, second result value) corresponding to the state of the subject by reflecting the first result value corresponding to the characteristic of the subject. The state of the subject may be the health state of the subject or the presence of a disease in the subject, and the second result value corresponding to the state of the subject may be a quantified value indicating the health state of the subject or the possibility of a specific disease in the subject.

According to an embodiment of the present disclosure, the second neural network model may be a model trained to output a second result value corresponding to the state of the subject by taking a biosignal as input. As an example, the second neural network model may comprise a model trained to output a second result value corresponding to a disease of the subject (a first sub-model of the second neural network model) and a model trained to output a second result value corresponding to a health state of the subject (a second sub-model of the second neural network model). The second neural network model may be implemented as a Multi-Layer Perceptron (MLP), a Convolutional Neural Network (CNN) model, a Recurrent Neural Network (RNN) model, a Relu model, or the like.

The processor 110 may input the acquired biosignal into the second neural network model to acquire a second result value corresponding to the state of the subject. Specifically, the second neural network model may extract feature information from the biosignal and output a second result value corresponding to the state of the subject based on the extracted feature information. For example, when the state of the subject is a disease of the subject and the disease to be predicted is left ventricular systolic dysfunction, the second neural network model may analyze the ECG signal, extract feature information (e.g., feature points related to left ventricular systolic dysfunction), and output a second result value by determining the possibility of left ventricular systolic dysfunction latent in the subject based on the extracted feature information. To this end, the second neural network model may be a model pre-trained based on training data comprising a plurality of input data including an ECG signal (or a feature-point-labeled ECG signal) and output data to which a label regarding left ventricular systolic dysfunction is assigned.

The second neural network model may be trained to output a second result value corresponding to the state of the subject through a transfer learning process based on the first neural network model. For example, the processor 110 may retrain the second neural network model through training data after transferring knowledge (e.g., weight values and parameter values of a plurality of layers constituting the network of the first neural network model) for extracting feature information from the ECG signal of the first neural network model trained to output the first result value corresponding to the characteristic of the subject to the second neural network model.

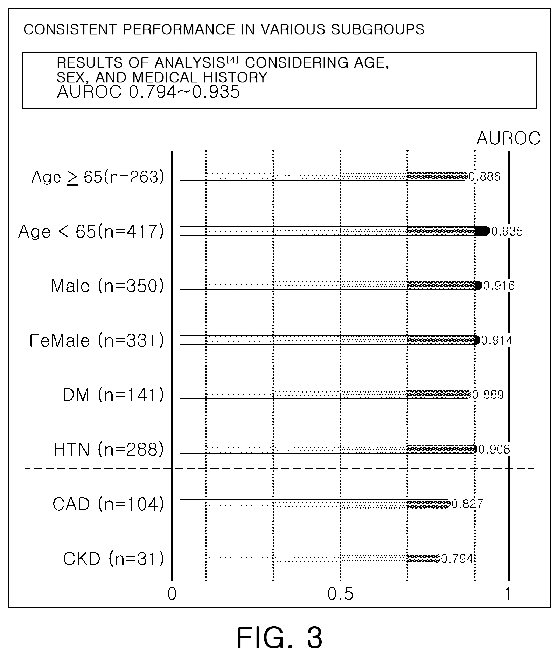

FIG. 3 is a diagram illustrating the accuracy of predicting left ventricular systolic dysfunction for different characteristics of subjects, according to an embodiment of the present disclosure.

Meanwhile, the accuracy of predicting the state of the subject may vary depending on the characteristic of the subject. Specifically, if the subject has a first characteristic, the accuracy of predicting the subject's disease may increase, or if the subject has a second characteristic, the accuracy of predicting the subject's disease may decrease. Referring to FIG. 3, the accuracy of the second neural network model trained to predict left ventricular systolic dysfunction is higher for a patient with hypertension with an AUROC of 0.908 than for a patient with chronic kidney disease with an AUROC of 0.794. As such, the accuracy of predicting a disease may vary depending on other characteristics or the presence of a disease in the patient other than the disease to be predicted.

Accordingly, according to an embodiment of the present disclosure, the processor 110 may adjust a performance indicator of the second neural network model based on the acquired first result value, and acquire a second result value corresponding to the state of the subject using the second neural network model with the adjusted performance indicator. Herein, the performance indicator may include at least one of a cut-off, a confidence, a sensitivity, and a specificity of the second neural network model. Specifically, when the processor 110 determines that a characteristic corresponding to the first result value exists in the subject based on the acquired first result value, it may adjust the performance of the second neural network model based on the relationship between the state prediction accuracy and the determined characteristic.

For example, if the characteristic corresponding to the first result value is hypertension, the processor 110 may adjust the performance of the second neural network model in a direction that increases the accuracy of the second result value of the second neural network model, in that the prediction accuracy of left ventricular systolic dysfunction of the second neural network model for a hypertensive patient is high. The processor 110 may adjust the cut-off of the second neural network model to be higher. Alternatively, the processor 110 may adjust the confidence of the second neural network model to be higher. Alternatively, the processor 110 may adjust the sensitivity of the second neural network model to be lower. Alternatively, the processor 110 may adjust the specificity of the second neural network model to be higher. Thereafter, the processor 110 may input the ECG signal into the second neural network model with the adjusted performance to acquire the second result value.

In addition, according to an embodiment of the present disclosure, the processor 110 may adjust a performance indicator of the second neural network model based on the acquired first result value and information about the environment in which the biosignal was acquired, and acquire a second result value corresponding to the state of the subject using the second neural network model with the adjusted performance indicator. Specifically, the processor 110 may adjust the performance of the second neural network model by considering the information about the environment in which the biosignal was acquired along with the presence or absence of the characteristic of the subject identified based on the acquired first result value.

For example, when the processor 110 determines that a characteristic corresponding to the first result value exists in the subject based on the first result value, it may primarily adjust the performance of the second neural network model based on the relationship between the state prediction accuracy and the determined characteristic, and secondarily adjust the performance of the second neural network model based on the environmental information. As an example, the environmental information may include the size of the hospital where the biosignal was measured, or whether the location where the biosignal was measured is an intensive care unit or a general health checkup center. In particular, the larger the size of the hospital where the biosignal was measured, or the higher the prevalence rate of the place where the biosignal was measured, such as an intensive care unit, the higher the accuracy of the biosignal and the lower the noise. This may lead to an increase in the accuracy of the second result value, which is the analysis result of the biosignal. Therefore, the processor 110 may adjust the second neural network model based on the information about the environment in which the biosignal was acquired. Since the adjustment of the second neural network model can be applied in the same way as the example described above, a detailed description thereof is omitted.

The processor 110 may predict the state of the subject based on the second result value (S240). As an example, when the second result value is equal to or greater than a preset value, the processor 110 may predict that the subject has a disease, and when the second result value is less than the preset value, it may predict that the subject does not have a disease.

Alternatively, the processor 110 may identify the health state of the subject corresponding to the second result value. Specifically, the processor 110 may divide the health state of the subject into a plurality of levels, set a range of the second result value corresponding to each level, and identify the health state of the subject with a level corresponding to the range in which the acquired second result value is included. For example, the processor 110 may set the health state of the subject to caution when the second result value is less than a first value, set the health state of the subject to frail when the second result value is equal to or greater than the first value and less than a second value, set the health state of the subject to good when the second result value is equal to or greater than the second value and less than a third value, and set the health state of the subject to healthy when the second result value is equal to or greater than the third value, thereby predicting the health state of the subject.

Hereinafter, an embodiment of the present disclosure for acquiring a second result value in which a first result value is reflected is described.

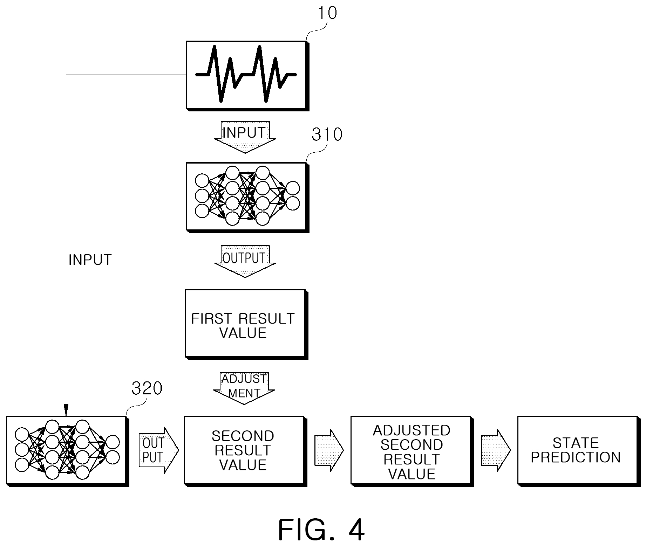

FIG. 4 is an exemplary diagram schematically illustrating a method for predicting a state of a patient based on a neural network model according to an embodiment of the present disclosure.

Referring to FIG. 4, according to an embodiment of the present disclosure, the processor 110 may adjust the second result value by applying a weight corresponding to the first result value acquired from the first neural network model 310 to the second result value. That is, the processor 110 may adjust the second result value output from the second neural network model 320 by reflecting the first result value corresponding to the characteristic of the subject. Thereafter, the processor 110 may predict the state of the subject based on the adjusted second result value.

The processor 110 may determine the value of the weight and the sign of the weight corresponding to the first result value to differ by the characteristic type of the subject. To this end, the processor 110 identifies the characteristic type of the subject and may determine the sign of the weight corresponding to the acquired first result value according to the characteristic type. Specifically, when the characteristic of the subject increases the accuracy of predicting the state, the processor 110 may determine the sign of the weight to be positive (+), and when the characteristic of the subject decreases the accuracy of predicting the state, it may determine the sign of the weight to be negative (-).

As an example, when the first result value corresponds to hypertension, the processor 110 may apply a positive sign to the weight corresponding to the acquired first result value, and when the first result value corresponds to chronic kidney disease, it may apply a negative sign to the weight corresponding to the acquired first result value.

For example, when the processor 110 acquires 1 as the first result value for the patient's hypertension by inputting the ECG signal into the first neural network model 310, and acquires 0.6 as the second result value for the possibility of left ventricular systolic dysfunction of the patient by inputting the ECG signal 10 into the second neural network model 320, the processor 110 may determine the sign of the weight corresponding to the first result value (1) to be positive (+). When the weight value corresponding to the first result value (1) is set to 20%, the processor 110 may acquire an adjusted second result value of 0.72 (0.6 + 0.6 x 20%) by applying the set weight value and the determined weight sign, yielding a determined weight (+20%), to the second result value (0.6). Meanwhile, when the reference value for the presence of left ventricular systolic dysfunction is 0.7, the processor 110 may determine that the adjusted second result value is equal to or greater than the reference value, and determine that the patient is predicted to have left ventricular systolic dysfunction.

On the other hand, when the processor 110 acquires 0.5 as the first result value for the patient's chronic kidney disease by inputting the ECG signal 10 into the first neural network model 310, and acquires 0.7 as the second result value for the possibility of left ventricular systolic dysfunction of the patient by inputting the ECG signal 10 into the second neural network model 320, the processor 110 may determine the sign of the weight corresponding to the first result value (0.5) to be negative (-). When the weight value corresponding to the first result value (0.5) is set to 10%, the processor 110 may acquire an adjusted second result value of 0.63 (0.7 - 0.7 x 10%) by applying the set weight value and the determined weight sign, yielding a determined weight (-10%), to the second result value (0.7). Meanwhile, when the reference value for the presence of left ventricular systolic dysfunction is 0.7, the processor 110 may determine that the adjusted second result value is less than the reference value, and determine that the patient is not predicted to have left ventricular systolic dysfunction.

Meanwhile, the processor 110 may determine the weight of the first result value by considering other characteristics of the subject together. The other characteristics of the subject may be identified through the biological information of the subject included in the ECG signal, such as the subject's age, sex, and the like, not through the first neural network model 310. For example, when the first result value corresponds to chronic kidney disease and the subject's age is equal to or less than a preset age, the processor 110 may determine the sign of the weight corresponding to the first result value to be negative.

Meanwhile, the processor 110 may also assign a confidence level to the second result value based on the first result value. This is to allow the user to estimate the accuracy of predicting the subject's state according to the second result value. Specifically, the processor 110 may calculate a confidence level for the second result value corresponding to the first result value and the characteristic type of the subject corresponding to the first result value, and assign it to the second result value. For example, when the first result value is the degree of possibility of chronic kidney disease and it is determined that the subject has chronic kidney disease based on the first result value, the processor 110 may calculate the confidence level of the second result value to be a low value. That is, the processor 110 may evaluate that the confidence of the accuracy of predicting the user's state based on the second result value is low, and provide related information as a confidence level.

Meanwhile, although the above example describes adjusting the second result value using the first result value corresponding to one characteristic, the second result value may also be adjusted using a plurality of first result values corresponding to a plurality of different characteristics.

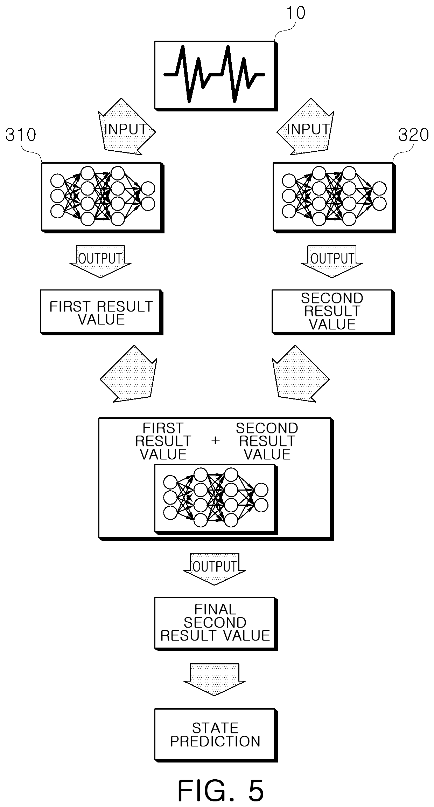

FIG. 5 is an exemplary diagram schematically illustrating a method for predicting a state of a patient by inputting a first result value and a second result value into a neural network model according to an embodiment of the present disclosure.

According to an embodiment of the present disclosure, the processor 110 may acquire a final result value corresponding to the state by inputting the first result value and the second result value into neural network models (i.e., the first neural network model 310 and the second neural network model 320), and predict the state of the subject based on the acquired final result value.

Specifically, referring to FIG. 5, the processor 110 may acquire a first result value by inputting an ECG signal 10 into the first neural network model 310, and may acquire a second result value by inputting the ECG signal 10 into the second neural network model 320. Thereafter, the processor 110 may input the acquired first result value and the second result value into a trained neural network model (hereinafter, third neural network model 330) that outputs a final result value corresponding to the state of the subject.

The third neural network model 330 may be a model trained to output a final result value corresponding to the state of the subject based on the second result value corresponding to the state of the subject and the first result value. The third neural network model 330 may be implemented as a Multi-Layer Perceptron (MLP), a Convolutional Neural Network (CNN) model, a Recurrent Neural Network (RNN) model, a Relu model, or the like. For example, when the state to be predicted is left ventricular systolic dysfunction and the subject has hypertension, the third neural network model 330 may combine the first result value for hypertension and the second result value for the possibility of left ventricular systolic dysfunction to output a final result value for the possibility of left ventricular systolic dysfunction. Meanwhile, the third neural network model 330 may be trained to output the final result value by considering the characteristic type of the subject in addition to the first result value and the second result value.

To this end, the third neural network model 330 may be a model pre-trained based on training data comprising a plurality of input data including the first result value and the second result value (and the characteristic type of the subject), and output data to which a label regarding left ventricular systolic dysfunction is assigned. Accordingly, the third neural network model 330 may combine the first result value and the second result value to output a final result value corresponding to the state. In this case, the processor 110 may predict the state of the subject based on the final result value. For example, if the state of the subject is a disease of the subject, the processor 110 may determine that the subject has the disease when the final result value is equal to or greater than a preset value, and determine that the subject does not have the disease when the final result value is less than the preset value.

Meanwhile, the third neural network model 330 may comprise a plurality of neural network models classified according to characteristic types. Therefore, the processor 110 may identify a characteristic type, select a neural network model corresponding to the identified characteristic type, and input the first result value and the second result value into the selected neural network model to acquire a final result value.

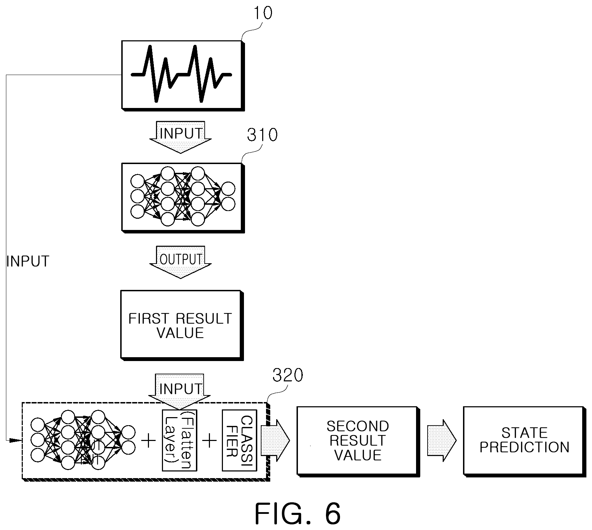

FIG. 6 is an exemplary diagram schematically illustrating a method for predicting a state of a patient by inputting a first result value and an ECG signal 10 into a second neural network model 320 according to an embodiment of the present disclosure.

According to an embodiment of the present disclosure, the processor 110 may acquire a second result value corresponding to the state of the subject by inputting the first result value corresponding to the characteristic of the subject together with the ECG signal 10 into the second neural network model 320. To this end, the second neural network model 320 may be trained to output a second result value corresponding to the state of the subject by combining the first result value with the biosignal. As an example, the first result value may be input to the flatten layer of the second neural network model 320.

Referring to FIG. 6, the processor 110 may input the first result value into the flatten layer while inputting the acquired ECG signal 10 into the second neural network model 320. Accordingly, by extracting the feature information of the ECG signal 10 from a convolutional layer before the flatten layer of the second neural network model 320, a one-dimensional vector corresponding to the extracted feature information may be output. Thereafter, the output one-dimensional vector and the first result value may be concatenated in the flatten layer and input to a classifier after the flatten layer. As a result, a final result value (i.e., a second result value) corresponding to the state is output from the output terminal of the second neural network model 320.

Meanwhile, the processor 110 may input the characteristic type of the subject into the second neural network model 320 along with the first and second result values. To this end, the second neural network model 320 may be trained to output a second result value based on the first result value, the biosignal, and the characteristic type. The first result value and the characteristic type may be input through the flatten layer of the second neural network model 320. Therefore, the processor 110 converts the characteristic type of the subject into a label value corresponding to the characteristic type of the subject and may input it into the flatten layer of the second neural network model 320 along with the first result value.

Meanwhile, according to an embodiment of the present disclosure, the second neural network model 320 may include a plurality of sub-neural network models corresponding to the characteristics of subjects. The plurality of sub-neural network models are classified according to characteristic types, and the plurality of sub-neural network models may be connected to a network part that extracts feature information of the biosignal in the second neural network model 320. Therefore, the processor 110 may identify a characteristic type, connect a sub-neural network model corresponding to the identified characteristic type to the network part that extracts feature information of the biosignal, and acquire a final result value.

In addition, according to an embodiment of the present disclosure, the processor 110 may acquire a noise score corresponding to the noise included in the biosignal as a first result value by inputting the acquired biosignal into the first neural network model. In this case, the processor 110 may acquire a second result value corresponding to the state of the subject by inputting the acquired noise score and the biosignal into the second neural network model 320. In addition, the second neural network model 320 may be trained to output a second result value corresponding to the state of the subject based on the acquired noise score and the biosignal. As an example, the first result value corresponding to the noise score may be input to the flatten layer of the second neural network model 320. Since this is the same as the description above, a detailed description is omitted.

According to an embodiment of the present disclosure, the processor 110 may determine whether to proceed with the state analysis of the subject based on the acquired noise score. Specifically, when the processor 110 determines that there is a lot of noise in the biosignal based on the noise score, it may decide not to proceed with the state analysis of the subject. That is, the processor 110 may not acquire the second result value based on the biosignal. This is because when there is a lot of noise in the biosignal, the accuracy of the second result value may decrease, so to secure the reliability of the prediction result, the processor 110 may determine whether to proceed with the state analysis of the subject according to the noise score. For example, when the noise score is equal to or greater than a preset score, the processor 110 may determine that there is a lot of noise in the biosignal and decide not to proceed with the state analysis of the subject.

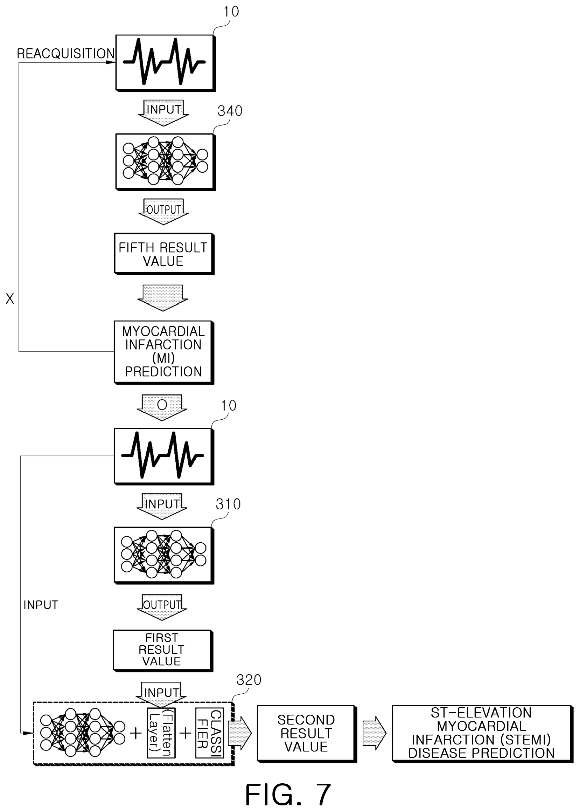

FIG. 7 is an exemplary diagram illustrating determination of whether to predict a first disease based on a prediction result of a second disease that is related to the first disease, according to an embodiment of the present disclosure.

According to an embodiment of the present disclosure, the processor 110 may acquire a third result value corresponding to another disease related to a disease included in the state of the subject by inputting the acquired biosignal into a neural network model (hereinafter, fourth neural network model) 340. Thereafter, when the processor 110 determines that the other disease exists based on the third result value, it may acquire a first result value corresponding to the characteristic based on the acquired biosignal and the first neural network model 310.

Specifically, the processor 110 may determine whether another disease related to the disease is present in the subject before predicting the disease. Hereinafter, for convenience of explanation of the invention, the disease to be predicted is referred to as a first disease, and the disease to be predicted in advance to predict the first disease, which is related to the first disease, is referred to as a second disease.

The processor 110 may acquire a third result value corresponding to the second disease by inputting the biosignal into the fourth neural network model 340. Herein, the third result value may be a quantified value indicating the degree of possibility of the second disease. As an example, the fourth neural network model 340 may be a model trained to calculate a third result value when an ECG signal 10 is input.

To this end, the fourth neural network model 340 may be a model pre-trained based on training data including a plurality of input data comprising an ECG signal 10 (or a feature-point-labeled ECG signal 10) and a plurality of output data to which a label regarding the second disease is assigned. The fourth neural network model 340 may be implemented as a Multi-Layer Perceptron (MLP), a Convolutional Neural Network (CNN) model, a Recurrent Neural Network (RNN) model, a Relu model, or the like.

As an example, referring to FIG. 7, the second disease may be myocardial infarction (MI), and the first disease may be ST-elevation myocardial infarction (STEMI). The processor 110 may acquire a fifth result value corresponding to MI by inputting the ECG signal 10 into the fourth neural network model 340, and if it is determined that the subject has MI based on the fifth result value, it may decide to predict STEMI based on the ECG signal 10.

Accordingly, the processor 110 may acquire a first result value by inputting the ECG signal 10 into the first neural network model 310, acquire a second result value using the acquired first result value and the second neural network model 320, and predict STEMI based on the acquired second result value. Meanwhile, if the processor 110 determines that the subject does not have MI based on the fifth result value, it may stop predicting STEMI based on the ECG signal 10. That is, the processor 110 may not acquire the first result value.

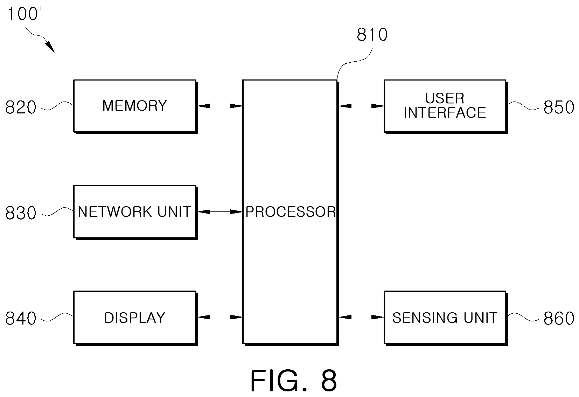

FIG. 8 is a detailed block diagram of a computing device 100' according to another embodiment of the present disclosure.

Referring to FIG. 8, the computing device 100' according to an embodiment of the present disclosure includes a processor 810, a memory 820, a network unit 830, a display 840, a user interface 850, and a sensing unit 860. For the components shown in FIG. 8 that overlap with the components shown in FIG. 2, detailed explanations are omitted.

The display 840 may display various images. Herein, the images include both still images and moving images. The display 140 may also output prediction results regarding the subject's disease. The display 140 may be implemented as various types of displays such as a liquid crystal display panel (LCD), an organic light-emitting diode (OLED), a liquid crystal on silicon (LCoS), or a digital light processing (DLP). In addition, the display 140 may also include a driving circuit, a backlight unit, and the like that can be implemented in the form of an a-Si TFT, a low temperature polysilicon (LTPS) TFT, an organic TFT (OTFT), or the like.

Meanwhile, the display 840 may be implemented as a touch screen by being combined with a touch panel, and the display 840 may perform the function of an input interface that receives a user's touch input as well as an output interface that outputs an image through the touch screen.

The user interface 850 is a component used for the computing device 100' to perform interaction with a user, and may include at least one of a touch sensor, a motion sensor, a button, a jog dial, and a switch, but is not limited thereto. The processor 810 may receive time series data through the user interface 850.

The sensing unit 860 senses the patient's biological information to acquire the patient's biosignal. As an example, the sensing unit 860 may acquire the patient's ECG signal 10. To this end, the sensing unit 860 may include at least one electrode and the like.

The various embodiments of the present disclosure described above may be combined with additional embodiments, and may be modified within the scope understandable by a person of ordinary skill in the art in light of the foregoing detailed description. The embodiments of the present disclosure are intended to be illustrative in all aspects and not restrictive. For example, each component described as a single unit may be implemented in a distributed manner, and likewise, components described as distributed may be implemented in a combined form. Therefore, all changes or modified forms derived from the meaning and scope of the claims of the present disclosure and their equivalent concepts are to be interpreted as being included in the scope of the present disclosure.

## Claims

1. A method for predicting a disease of a patient based on a neural network model, performed by a computing device including one or more processors, comprising:
acquiring a biosignal of a subject;
acquiring a first result value corresponding to a characteristic of the subject or a characteristic of the biosignal, based on the acquired biosignal and a first neural network model;
acquiring a second result value corresponding to a state of the subject, based on the acquired first result value and a second neural network model; and
predicting the state of the subject based on the second result value.

2. The method of claim 1,
wherein the acquiring of the second result value comprises:
acquiring a second result value corresponding to the state of the subject by inputting the acquired biosignal into the second neural network model; and
acquiring an adjusted second result value by applying a weight corresponding to the acquired first result value to the second result value,
and wherein the predicting of the state of the subject comprises:
predicting the state of the subject based on the adjusted second result value.

3. The method of claim 3,
wherein the acquiring of the second result value comprises:
identifying a characteristic type of the subject and determining a sign of the weight corresponding to the acquired first result value according to the characteristic type.

4. The method of claim 4,
wherein:
the state of the subject comprises left ventricular systolic dysfunction;
the characteristic of the subject comprises hypertension and chronic kidney disease of the subject; and
the determining of the weight comprises applying a positive sign to the weight corresponding to the acquired first result value when the first result value corresponds to the hypertension, and applying a negative sign to the weight corresponding to the acquired first result value when the first result value corresponds to the chronic kidney disease.

5. The method of claim 1,
wherein the acquiring of the first result value comprises:
acquiring the first result value corresponding to the characteristic of the subject by inputting the acquired biosignal into the first neural network model,
and wherein the acquiring of the second result value comprises:
acquiring the second result value corresponding to the state of the subject by inputting the acquired first result value and the acquired biosignal into the second neural network model, the second neural network model trained to output the second result value corresponding to the state of the subject based on the first result value and the biosignal.

6. The method of claim 1, comprising:
acquiring a third result value corresponding to the state of the subject by inputting the acquired biosignal into a third neural network model,
wherein the acquiring of the first result value comprises:
acquiring the first result value corresponding to the characteristic of the subject by inputting the acquired biosignal into the first neural network model,
and wherein the acquiring of the second result value comprises:
acquiring the second result value corresponding to the state of the subject by inputting the acquired first result value and the acquired third result value into the second neural network model, the second neural network model trained to output the second result value corresponding to the state of the subject based on the third result value corresponding to the state of the subject and the first result value.

7. The method of claim 1, comprising:
acquiring a fourth result value corresponding to another disease related to a disease included in the state by inputting the acquired biosignal into a fourth neural network model,
wherein the acquiring of the first result value corresponding to the characteristic of the subject comprises:
acquiring the first result value corresponding to the characteristic based on the acquired biosignal and the first neural network model in response to a determination based on the fourth result value that the other disease exists.

8. The method of claim 1,
wherein:
the characteristic of the biosignal comprises noise included in the acquired biosignal;
the acquiring of the first result value comprises acquiring a noise score corresponding to the amount of the noise included in the biosignal as the first result value by inputting the acquired biosignal into the first neural network model; and
the acquiring of the second result value comprises acquiring the second result value corresponding to the state of the subject by inputting the acquired noise score and the biosignal into the second neural network model.

9. The method of claim 8, comprising:
terminating a state prediction process of the subject without acquiring the second result value when the noise score is equal to or greater than a preset value.

10. The method of claim 1, comprising:
adjusting a performance indicator of the second neural network model based on the acquired first result value, the performance indicator comprising at least one of a cut-off, a confidence, a sensitivity, and a specificity of the second neural network model; and
acquiring the second result value corresponding to the state of the subject using the second neural network model with the adjusted performance indicator.

11. The method of claim 10,
wherein the adjusting comprises:
adjusting the performance indicator of the second neural network model based on the acquired first result value and information about the environment in which the biosignal was acquired.

12. A computing device for predicting a disease of a patient
based on a neural network model, comprising:
a processor including one or more cores;
a memory including program codes executable by the processor; and
a network unit,
wherein the processor is configured to:
acquire a biosignal of a subject via the network unit; acquire a first result value corresponding to a characteristic of the subject based on the acquired biosignal and a first neural network model; acquire a second result value corresponding to a state of the subject based on the acquired first result value and a second neural network model; and predict the state of the subject based on the second result value.

13. A computer program stored in a computer-readable storage medium, the computer program, when executed by one or more processors, causing operations to be performed for predicting a disease of a patient based on a neural network model,
the operations comprising:
acquiring a biosignal of a subject;
acquiring a first result value corresponding to a characteristic of the subject based on the acquired biosignal and a first neural network model;
acquiring a second result value corresponding to a state of the subject based on the acquired first result value and a second neural network model; and
predicting the state of the subject based on the second result value.
